# EUROPÄISCHE PATENTSCHRIFT

(11) **EP 3 843 661 B1**
(45) Veröffentlichungstag und Bekanntmachung des Hinweises auf die Patenterteilung: **02.04.2025**
(21) Anmeldenummer: 19790177.0
(22) Anmeldetag: 16.10.2019
(51) Int. Cl.: A61F 2/12

(54) **VERFAHREN ZUR HERSTELLUNG EINER BRUSTPROTHESE MIT ANPASSBAREM VOLUMEN**
METHOD FOR PRODUCING A BREAST PROSTHESIS HAVING AN ADJUSTABLE VOLUME
PROCÉDÉ DE FABRICATION D'UNE PROTHÈSE MAMMAIRE À VOLUME ADAPTABLE

(30) Priorität: 18.10.2018 DE 102018125897
(43) Veröffentlichungstag der Anmeldung: 07.07.2021
(73) Patentinhaber: Amoena Medizin-Orthopädie-Technik GmbH, 83064 Raubling (DE)
(72) Erfinder: STELTER, Nils, 83112 Frasdorf (DE); WILD, Helmut, 83074 Stephanskirchen (DE)
(74) Vertreter: Laufhütte, Dieter
(86) Internationale Anmeldenummer: PCT/EP2019/078042
(87) Internationale Veröffentlichungsnummer: WO 2020/079049

(56) Entgegenhaltungen:
- EP-A1- 2 944 291
- EP-A1- 3 243 487
- WO-A1-2011/098283
- WO-A1-2016/109117

## Beschreibung

Die Erfindung betrifft ein Verfahren zur Herstellung einer Brustprothese bzw. Brust-Epithese mit anpassbarem Volumen.

Brustprothesen werden nach operativen Brustentfernungen getragen. Anforderungen an die Brustprothesen umfassen insbesondere eine der natürlichen Brust möglichst nahekommenden Form und Haptik sowie ein hoher Tragekomfort.

Verfahen zur Herstellung Brustprosthesen sind z.B. aus EP 2944291, EP 3243487 und WO 2016/109117 bekannt.

Um ohne kostspielige Individualanfertigungen erreichen zu können, dass das Volumen einer derartigen Prothese an die individuellen Bedürfnisse der Trägerin angepasst werden kann, die sich aus der Größe der noch gesunden Brust, sofern vorhanden, oder aus dem persönlichen Wohlbefinden ableiten, wurde bereits im Stand der Technik vorgeschlagen, Brustprothesen bereitzustellen, die in ihrem Volumen nachträglich angepasst werden können. Als Beispiel hierfür ist etwa die EP 0 824 001 A2 zu nennen.

Aufgabe der Erfindung ist es, ein Verfahren zur Herstellung einer volumenanpassbaren Brustprothese mit verbesserten Eigenschaften bereitzustellen.

Vor diesem Hintergrund betrifft die Erfindung ein Verfahren zur Herstellung einer Brustprothese mit anpassbarem Volumen, wobei die Brustprothese einen ersten Schalenkörper, einen damit umlaufend verbundenen zweiten Schalenkörper und einen zwischen den Schalenkörpern angeordneten Fluidraum aufweist, wobei das Verfahren die folgenden Schritte aufweist: (a) umfängliches Verbinden von vier Kunststofffolien zur Bereitstellung eines Templates umfassend drei Kammern; (b) Befüllen der oberen und unteren Kammer mit einer vernetzbaren Silikonmasse; und (c) Vernetzen der Silikonmasse.

Bei den Schalenkörpern der so hergestellten Brustprothese handelt es sich also um mit einem verformbaren Material gefüllte Folienbeutel. Bei der vernetzbaren Silikonmasse handelt es sich vorzugsweise um eine Zwei-Komponenten-Silikonmasse. Nach dem Vernetzen resultiert aus einer derartigen Masse ein quervernetzter Silikonkautschuk, vorzugsweise Zwei-Komponenten-Silikonkautschuk. Dieser Kautschuk erlaubt es den Schalenkörpern die Anforderungen an Haptik und Tragekomfort zu erfüllen und eine Expansion des Fluidraumvolumens nicht zu behindern.

Die vernetzbare Silikonmasse kann zusätzliche Bestandteile wie beispielsweise ein Phasenwechselmaterial oder poröses Granulat bzw. Hohlkugeln umfassen. Phasenwechselmaterialien dienen zur Verbesserung der Wärmeregulierung an der Haut der Trägerin und werden daher bevorzugt der Silikonmasse des ersten Schalenkörpers beigemengt, der in der Anwendung auf Seiten der Trägerin liegen soll. Geeignete Phasenwechselmaterialien umfassen solche, deren Phasenübergangstemperatur nahe der Körpertemperatur liegt. Beispiele umfassen Paraffine mit einer geeigneten Zahl an Kohlenstoffatomen, typischerweise etwa zwanzig, um einen Schmelzpunkt im gewünschten Bereich einzustellen. Granulate bzw. Hohlkugeln können dazu dienen, das Gewicht der Prothese zu verringern, ohne die haptischen Eigenschaften zu beeinträchtigen.

Die Angaben von vier Folien und drei Kammern sind im Sinne einer Mindestangabe zu verstehen. Auch Varianten mit mehr als drei Kammern sind von der Erfindung umfasst, was die Verbindung von mehr als vier Folien erfordern würde.

Bei dem umfänglichen Verbinden der vier Kunststofffolien gemäß Schritt (a) kann es sich um ein umfängliches Verschweißen handeln. Alternativ oder zusätzlich könnte auch ein Verkleben entlang der gemeinsamen Umfangsfläche erfolgen.

Es kann vorgesehen sein, dass im Rahmen des umfänglichen Verbindens der vier Kunststofffolien gemäß Schritt (a) zwischen den beiden oberen und/oder zwischen den beiden unteren Folien eine Unterbrechung freigelassen wird, um einen Zugang in die jeweilige Kammer zu bilden. Das Befüllen der oberen und/oder unteren Kammer gemäß Schritt (b) kann durch die Unterbrechung erfolgen.

Vorzugsweise ist vorgesehen dass das Vernetzen der Silikonmasse durch Temperaturerhöhung erfolgt.

Die Unterbrechung wird vor oder nach dem Vernetzen geschlossen, vorzugsweise verschweißt.

Im Rahmen des umfänglichen Verbindens der vier Kunststofffolien gemäß Schritt (a) wird ein Ventilschlauch zwischen den beiden mittleren Folien eingeschlossen, um einen radialen Zugang zum Fluidraum zu bilden. Der Schlauch wird zwischen den Folien eingeklebt oder eingeschweißt und kann die Schweiß- oder Klebenaht zwischen den Folienbeuteln so in radiale Richtung durchdringen. Die so hergestellte Brustprothese umfasst also ferner einen Ventilschlauch aus einem flexiblen Material und vorzugsweise Kunststoffmaterial, der im Verbindungsbereich zwischen den Schalenkörpern von außen in den Fluidraum reicht und über den Verbindungsbereich hinaus in den Fluidraum ragt. Der Ventilschlauch weist generell ein Rückschlagventil wie beispielsweise ein Flatterventil auf und dient dazu, um eine nachträgliche, d.h. nach der Herstellung erfolgende Befüllung und Entleerung des Fluidraums zu ermöglichen.

Es kann vorgesehen sein, dass das Verfahren zusätzlich einen Schritt der Einbringung eines Mediums zur Verringerung der Adhäsionsneigung der gegenüberliegenden Innenoberflächen der Schalenkörper in den Fluidraums umfasst, wobei vorzugsweise vorgesehen ist, dass das Medium zur Verringerung der Adhäsionsneigung der gegenüberliegenden Innenoberflächen der Schalenkörper durch den Ventilschlauch in den Fluidraum eingebracht wird. Beispielsweise kann eine Nadel in den Ventilschlauch gesteckt werden und das Medium durch diese Nadel in die Kammer gedrückt werden.

Eine derart hergestellte Brustprothese umfasst in dem Fluidraum, dessen Befüllung mit einem Fluid zur Volumenanpassung beabsichtigt ist, ein von dem dem Fluid zur Volumenanpassung verschiedenes, zusätzliches Medium, welches die Adhäsionsneigung der gegenüberliegenden Innenoberflächen der Schalenkörper verringert. Die nachträgliche Volumenanpassung kann dadurch behindert werden, dass die Innenoberflächen der Schalenkörper aneinander haften bleiben. Auch eine permanente Deformierung der Brustprothese durch ein derartiges Anhaften wäre zu befürchten. Deshalb sieht die Erfindung vor, die Tendenz für eine solche unerwünschte Adhäsion zu verringern.

Bei dem Medium zur Verringerung der Adhäsionsneigung der gegenüberliegenden Innenoberflächen der Schalenkörper kann es sich um eine Flüssigkeit, insbesondere um ein Öl und weiter bevorzugt um ein Silikon-Öl handeln. In dem Fall, dass es sich sowohl bei dem Fluid zur Volumenanpassung als auch bei dem Medium zur Verringerung der Adhäsionsneigung der gegenüberliegenden Innenoberflächen der Schalenkörper jeweils um eine Flüssigkeit handelt, unterscheiden sich diese Flüssigkeiten voneinander.

Ferner kann es sich bei dem Medium zur Verringerung der Adhäsionsneigung der gegenüberliegenden Innenoberflächen der Schalenkörper um einen pulverförmigen Feststoff handeln. Die durchschnittliche Partikelgröße der Pulverkörner liegt vorzugsweise im Bereich von zwischen 1 nm bis 1 mm. Das Pulver kann beispielsweise in Kombination mit einem Gas in den Fluidraum eingeblasen werden.

Bei dem Medium zur Verringerung der Adhäsionsneigung der gegenüberliegenden Innenoberflächen der Schalenkörper kann es sich gegebenenfalls auch um ein Gas handeln. In dem Fall, dass es sich sowohl bei dem Fluid zur Volumenanpassung als auch bei dem Medium zur Verringerung der Adhäsionsneigung der gegenüberliegenden Innenoberflächen der Schalenkörper jeweils um ein Gas handelt, unterscheiden sich diese Gase voneinander.

Auch Kombinationen aus den genannten Medien zur Verringerung der Adhäsionsneigung der gegenüberliegenden Innenoberflächen der Schalenkörper kommen in weiteren Ausführungsformen zur Anwendung. Beispielsweise kann ein Gemisch aus Flüssigkeiten und dispergierten pulverförmigen Feststoffen verwendet werden, wobei die Flüssigkeiten und dispergierten pulverförmigen Feststoffe wie oben definiert ausgebildet sein können.

In einer Variante ist vorgesehen, dass das Medium zur Verringerung der Adhäsionsneigung der gegenüberliegenden Innenoberflächen der Schalenkörper vor dem Befüllen der oberen und unteren Kammer mit einer vernetzbaren Silikonmasse gemäß Schritt (b) eingebracht wird.

In einer alternativen Variante ist vorgesehen, dass das Medium zur Verringerung der Adhäsionsneigung der gegenüberliegenden Innenoberflächen der Schalenkörper zwischen dem Befüllen der oberen und unteren Kammer mit einer vernetzbaren Silikonmasse gemäß Schritt (b) und dem Vernetzen der Silikonmasse gemäß Schritt (c) eingebracht wird.

In einer wiederum anderen Variante ist vorgesehen, dass das Medium zur Verringerung der Adhäsionsneigung der gegenüberliegenden Innenoberflächen der Schalenkörper nach dem Vernetzen der Silikonmasse gemäß Schritt (c) eingebracht wird. Der Schritt (c) sowie gegebenenfalls der Schritt (a) und/oder die Einbringung des Mediums zur Verringerung der Adhäsionsneigung der gegenüberliegenden Innenoberflächen der Schalenkörper erfolgt vorzugsweise in einem formgebenden Werkzeug. In diesem formgebenden Werkzeug kann auch das Schließen, vorzugsweise Verschweißen der Durchlässe zum Einbringen der Silikonmasse in die werdenden Schalenkörper erfolgen.

Es ist beabsichtigt, dass das Volumen der Brustprothese nachträglich, d.h. anwenderseitig durch Einfüllen von Fluid zur Volumenanpassung in den Fluidraum angepasst wird. Bei dem Fluid zur Volumenanpassung kann es sich um ein Gas, insbesondere um Luft handeln. Ferner kann es sich bei dem Fluid zur Volumenanpassung um eine Flüssigkeit, insbesondere um eine nachträglich vernetzbare viskose Flüssigkeit und weiter vorzugsweise um ein vernetzbares Silikon-Fluid handeln. Auch Kombinationen sind denkbar.

Weitere Einzelheiten und Vorteile der Erfindung ergeben sich aus dem nachfolgend anhand der Figuren beschriebenen Ausführungsbeispiel. In den Figuren zeigen:
- Figur 1:: eine schematische Darstellung einer anhand des erfindungsgemäßen Verfahrens hergestellten volumenanpassbaren Brustprothese;
- Figur 2:: einen Ablauf einer Ausführungsvariante eines erfindungsgemäßen Verfahrens;
- Figur 3:: einen Ablauf einer weiteren Ausführungsvariante eines erfindungsgemäßen Verfahrens; und
- Figur 4:: einen Ablauf einer wiederum anderen Ausführungsvariante eines erfindungsgemäßen Verfahrens.

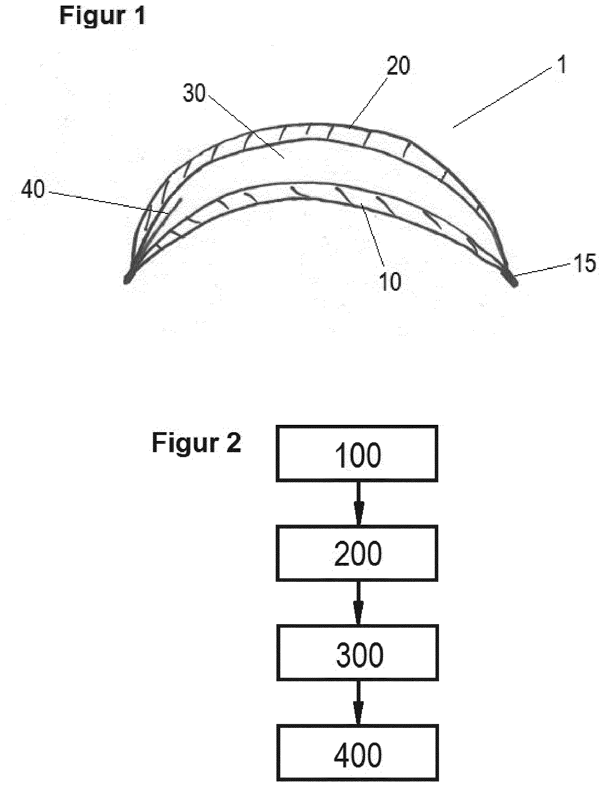

Die in Figur 1 dargestellte Brustprothese 1 mit anpassbarem Volumen kann anhand eines erfindungsgemäßen Verfahrens hergestellt werden. Die Brustprothese 1 umfasst einen ersten Schalenkörper 10 an der der Trägerin zugewandten Unterseite der Prothese 1 sowie einen damit umlaufend verbundenen zweiten Schalenkörper 20 an der der Trägerin abgewandten Oberseite der Prothese 1. Bei beiden Schalenkörpern 10 und 20 handelt es sich um Folienbeutel, die mit einer quervernetzten Zwei-Komponenten-Silikonkautschuk-Masse gefüllt sind. Die Folienbeutel sind jeweils aus zwei Kunststofffolienstücken gefertigt, die entlang der gemeinsamen Umfangsfläche miteinander verschweißt sind.

Die Schalenkörper 10 und 20 sind ihrerseits entlang einer umlaufenden Schweißnaht 15 derart verbunden, dass zwischen ihnen ein Fluidraum 30 gebildet wird, der beispielsweise mit Luft, aber auch mit einer Flüssigkeit gefüllt werden kann. Das Volumen der Brustprothese 1 kann durch Befüllung und Entleerung des Fluidraums 30 angepasst werden.

Um eine nachträgliche, d.h. nach der Herstellung erfolgende Befüllung und Entleerung des Fluidraums 30 zu ermöglichen, umfasst die Brustprothese 1 einen Ventilschlauch 40 aus einem flexiblen Kunststoffmaterial, der ein flaches Flatterventil umfasst, die Schweißnaht 15 in radiale Richtung durchdringt und dabei zwischen den Schalenkörpern 10 und 20 eingeschweißt ist. Der Ventilschlauch 40 reicht nicht lediglich bis zum Ende der Schweißnaht 15, sondern ragt frei, d.h. ohne mit einem der Schalenkörper 10 oder 20 verbunden zu sein, über die Schweißnaht 15 hinaus in den Fluidraum 30 hinein. Der in den Fluidraum 30 ragende Abschnitt des Ventilschlauchs 40 ist also frei im Fluidraum 30 beweglich.

Um ein Zusammenhaften der Kunststoff-Innenoberflächen der beiden Schalenkörper 10 und 20 aufgrund beispielsweise elektrostatischer Wechselwirkung und mithin ein unerwünschtes Verkleben des Fluidraums 30 zu verhindern, ist der Fluidraum 30 mit einer kleineren Menge an Silikon-Öl befüllt.

In Figuren 2-4 sind drei Varianten möglicher Abläufe eines erfindungsgemäßen Verfahrens zur Herstellung der in Figur 1 gezeigten Brustprothese 1 schematisch dargestellt.

Allen Verfahren ist gemein, dass in einem ersten Schritt 100, entsprechend dem Schritt (a), vier Kunststofffolien zur Bereitstellung eines Templates umfassend drei Kammern umfänglich verbunden werden.

In einer ersten Variante der Verfahrensführung, wie gezeigt in Figur 2, folgt hierauf unmittelbar ein Schritt 200, entsprechend dem Schritt (b), wobei die obere Kammer, d.h. die Kammer zwischen den oberen beiden Folien, und die untere Kammer, d.h. die Kammer zwischen den oberen beiden Folien, mit einer vernetzbaren Zwei-Komponenten-Silikonmasse befüllt werden. Sodann erfolgt das Aushärten durch Wärmeeinwirkung gemäß Schritt 300, entsprechend dem Schritt (c). Erst im Anschluss an diesen Schritt erfolgt in einem letzten Schritt 400 das Einbringen des Mediums zur Verringerung der Adhäsionsneigung der gegenüberliegenden Innenoberflächen der Schalenkörper.

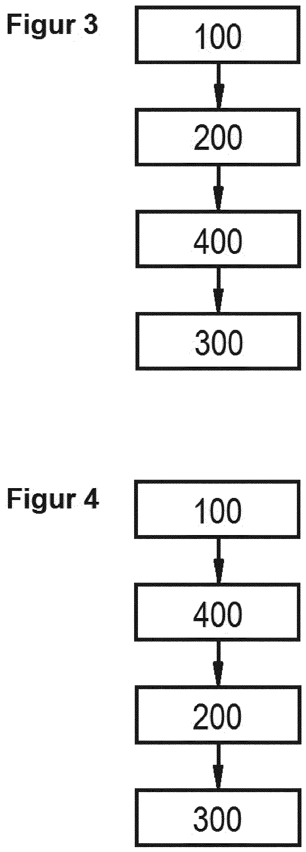

In einer zweiten Variante der Verfahrensführung, wie gezeigt in Figur 3, folgt auf den Schritt 100 weiterhin unmittelbar der Schritt 200, wobei das Medium zur Verringerung der Adhäsionsneigung der gegenüberliegenden Innenoberflächen der Schalenkörper gemäß Schritt 400 jedoch vor dem Aushärten durch Wärmeeinwirkung gemäß Schritt 300, entsprechend dem Schritt (c) erfolgt.

In einer dritten Variante der Verfahrensführung, wie gezeigt in Figur 4, folgt auf den Schritt 100 direkt die Einbringung des Mediums zur Verringerung der Adhäsionsneigung der gegenüberliegenden Innenoberflächen der Schalenkörper gemäß Schritt 400. Erst im Anschluss wird gemäß Schritten 200 und 300 die Silikonmasse in die oberen und unteren Kammern eingefüllt und ausgehärtet.

Die Aushärtung der Silikonmasse gemäß Schritt 300 erfolgt dabei jeweils in einem formgebenden Werkzeug unter Temperatureinwirkung. Die Durchgänge in den Schweißnähten, durch welche die Silikonmassen in die oberen und unteren Kammern eingefüllt werden, können in diesem formgebenden Werkzeug verschweißt werden, entweder vor oder nach dem Aushärten.

## Patentansprüche

1. Verfahren zur Herstellung einer Brustprothese mit nachträglich anwenderseitig anpassbarem Volumen, wobei die Brustprothese einen ersten Schalenkörper (10), einen damit umlaufend verbundenen zweiten Schalenkörper (20) und einen zwischen den Schalenkörpern angeordneten Fluidraum (30) aufweist, der anwenderseitig durch Einfüllen von Fluid an ein gewünschtes Volumen anpassbar ist, wobei das Verfahren die folgenden Schritte aufweist:
(a) umfängliches Verbinden von vier Kunststofffolien zur Bereitstellung eines Templates umfassend drei Kammern;
(b) Befüllen der oberen und unteren Kammer mit einer vernetzbaren Silikonmasse; und
(c) Vernetzen der Silikonmasse
**dadurch gekennzeichnet,**
**dass** im Rahmen des umfänglichen Verbindens der vier Kunststofffolien gemäß Schritt (a) ein Ventilschlauch (40) zwischen den beiden mittleren Folien eingeschlossen wird, um einen radialen Zugang zum Fluidraum zu bilden.

2. Verfahren nach Anspruch 1, **dadurch gekennzeichnet, dass** es sich bei dem umfänglichen Verbinden der vier Kunststofffolien gemäß Schritt (a) um ein umfängliches Verschweißen handelt.

3. Verfahren nach einem der vorhergehenden Ansprüche, **dadurch gekennzeichnet, dass** im Rahmen des umfänglichen Verbindens der vier Kunststofffolien gemäß Schritt (a) zwischen den beiden oberen und/oder zwischen den beiden unteren Folien eine Unterbrechung freigelassen wird, um einen Zugang in die jeweilige Kammer zu bilden.

4. Verfahren nach Anspruch 3, **dadurch gekennzeichnet, dass** das Befüllen der oberen und/oder unteren Kammer gemäß Schritt (b) durch die Unterbrechung erfolgt.

5. Verfahren nach einem der vorhergehenden Ansprüche, **dadurch gekennzeichnet, dass** das Vernetzen der Silikonmasse durch Temperaturerhöhung erfolgt.

6. Verfahren nach einem der vorhergehenden Ansprüche, **dadurch gekennzeichnet, dass** das Verfahren zusätzlich einen Schritt der Einbringung eines Mediums zur Verringerung der Adhäsionsneigung der gegenüberliegenden Innenoberflächen der Schalenkörper (10, 20) in den Fluidraum (30) umfasst, wobei vorzugsweise vorgesehen ist, dass das Medium zur Verringerung der Adhäsionsneigung der gegenüberliegenden Innenoberflächen der Schalenkörper (10, 20) durch den Ventilschlauch (40) in den Fluidraum (30) eingebracht wird.

7. Verfahren nach Anspruch 6, **dadurch gekennzeichnet, dass** das Medium zur Verringerung der Adhäsionsneigung der gegenüberliegenden Innenoberflächen der Schalenkörper vor dem Befüllen der oberen und unteren Kammer mit einer vernetzbaren Silikonmasse gemäß Schritt (b) eingebracht wird.

8. Verfahren nach Anspruch 6, **dadurch gekennzeichnet, dass** das Medium zur Verringerung der Adhäsionsneigung der gegenüberliegenden Innenoberflächen der Schalenkörper (10, 20) zwischen dem Befüllen der oberen und unteren Kammer mit einer vernetzbaren Silikonmasse gemäß Schritt (b) und dem Vernetzen der Silikonmasse gemäß Schritt (c) eingebracht wird.

9. Verfahren nach Anspruch 6, **dadurch gekennzeichnet, dass** das Medium zur Verringerung der Adhäsionsneigung der gegenüberliegenden Innenoberflächen der Schalenkörper (10, 20) nach dem Vernetzen der Silikonmasse gemäß Schritt (c) eingebracht wird.

## Claims

1. Method of producing a breast prosthesis having a volume subsequently adjustable by the user, wherein the breast prosthesis comprises a first shell body (10), a second shell body (20) peripherally connected thereto, and a fluid space (30) arranged between the shell bodies, which space can be adjusted by the user to a desired volume by filling it with liquid, wherein the method comprises the following steps:
(a) peripherally connecting four plastic films to provide a template comprising three chambers;
(b) filling the upper and lower chambers with a crosslinkable silicone compound; and
(c) crosslinking the silicone compound,
**characterized in that**
a valve tube (40) is enclosed between the two middle films to form radial access to the fluid space in the course of the peripheral connection of the four plastic films in accordance with step (a).

2. Method in accordance with claim 1, **characterized in that** the peripheral connection of the four plastic films in accordance with step (a) is a peripheral welding.

3. Method in accordance with one of the preceding claims, **characterized in that**, to form access to the respective chamber, an interruption is left free between the two upper films and/or between the two lower films in the course of the peripheral connection of the four plastic films in accordance with step (a).

4. Method in accordance with claim 3, **characterized in that** the filling of the upper and/or lower chambers in accordance with step (b) takes place through the interruption.

5. Method in accordance with one of the preceding claims, **characterized in that** the crosslinking of the silicone compound takes place by increasing temperature.

6. Method in accordance with one of the preceding claims, **characterized in that** the method additionally comprises a step of introducing a medium for reducing the adhesive tendency of the oppositely disposed inner surfaces of the shell bodies (10, 20) into the fluid space (30), with provision preferably being made that the medium for reducing the adhesive tendency of the oppositely disposed inner surfaces of the shell bodies (10, 20) is introduced into the fluid space (30) through the valve tube (40).

7. Method in accordance with claim 6, **characterized in that** the medium for reducing the adhesive tendency of the oppositely disposed inner surfaces of the shell bodies is introduced before the filling of the upper and lower chambers with a crosslinkable silicone compound in accordance with step (b).

8. Method in accordance with claim 6, **characterized in that** the medium for reducing the adhesive tendency of the oppositely disposed inner surfaces of the shell bodies (10, 20) is introduced between the filling of the upper and lower chambers with a crosslinkable silicone compound in accordance with step (b) and the crosslinking of the silicone compound in accordance with step (c).

9. Method in accordance with claim 6, **characterized in that** the medium for reducing the adhesive tendency of the oppositely disposed inner surfaces of the shell bodies (10, 20) is introduced after the crosslinking of the silicone compound in accordance with step (c).

## Revendications

1. Procédé de fabrication d'une prothèse mammaire avec un volume adaptable ultérieurement par l'utilisateur, la prothèse mammaire présentant un premier corps de coque (10), un deuxième corps de coque (20) relié périphériquement à celui-ci et une chambre de fluide (30) agencée entre les corps de coque, qui est adaptable par l'utilisateur à un volume souhaité par remplissage de fluide, le procédé présentant les étapes suivantes :
(a) la liaison circonférentielle de quatre films de matière plastique pour fournir un gabarit comprenant trois chambres ;
(b) le remplissage des chambres supérieure et inférieure avec une masse de silicone réticulable ; et
(c) la réticulation de la masse de silicone,
**caractérisé en ce que**
dans le cadre de la liaison circonférentielle des quatre films de matière plastique selon l'étape (a), un tuyau de valve (40) est enfermé entre les deux films centraux pour former un accès radial à la chambre de fluide.

2. Procédé selon la revendication 1, **caractérisé en ce que** la liaison circonférentielle des quatre films de matière plastique selon l'étape (a) consiste en un soudage circonférentiel.

3. Procédé selon l'une quelconque des revendications précédentes, **caractérisé en ce que**, dans le cadre de la liaison circonférentielle des quatre films de matière plastique selon l'étape (a), une interruption est laissée libre entre les deux films supérieurs et/ou entre les deux films inférieurs pour former un accès à la chambre respective.

4. Procédé selon la revendication 3, **caractérisé en ce que** le remplissage des chambres supérieure et/ou inférieure selon l'étape (b) est effectué à travers l'interruption.

5. Procédé selon l'une quelconque des revendications précédentes, **caractérisé en ce que** la réticulation de la masse de silicone est effectuée par augmentation de la température.

6. Procédé selon l'une quelconque des revendications précédentes, **caractérisé en ce que** le procédé comprend en outre une étape d'introduction dans la chambre de fluide (30) d'un milieu destiné à réduire la tendance à l'adhésion des surfaces intérieures opposées des corps de coque (10, 20), il étant de préférence prévu que le milieu destiné à réduire la tendance à l'adhésion des surfaces intérieures opposées des corps de coque (10, 20) est introduit dans la chambre de fluide (30) par le tuyau de valve (40).

7. Procédé selon la revendication 6, **caractérisé en ce que** le milieu destiné à réduire la tendance à l'adhésion des surfaces intérieures opposées des corps de coque est introduit avant le remplissage des chambres supérieure et inférieure avec une masse de silicone réticulable selon l'étape (b).

8. Procédé selon la revendication 6, **caractérisé en ce que** le milieu destiné à réduire la tendance à l'adhésion des surfaces intérieures opposées des corps de coque (10, 20) est introduit entre le remplissage des chambres supérieure et inférieure avec une composition de silicone réticulable selon l'étape (b) et la réticulation de la composition de silicone selon l'étape (c).

9. Procédé selon la revendication 6, **caractérisé en ce que** le milieu destiné à réduire la tendance à l'adhésion des surfaces intérieures opposées des corps de coque (10, 20) est introduit après la réticulation de la masse de silicone selon l'étape (c).
